# EUROPEAN PATENT SPECIFICATION

(11) **EP 2 940 028 B1**
(45) Date of publication and mention of the grant of the patent: **16.08.2017**
(21) Application number: 15161799.0
(22) Date of filing: 06.01.2004
(51) Int. Cl.: C07H 15/04, C07H 5/02, C07H 11/04, C07H 13/04

(54) **CERTAIN AMINOALKYL GLUCOSAMINIDE PHOSPHATE COMPOUNDS AND THEIR USE**
BESTIMMTE AMINOALKYL-GLUCOSAMINID-PHOSPHATVERBINDUNGEN UND IHRE VERWENDUNG
COMPOSES D'AMINOALKYLE GLUCOSAMINIDE PHOSPHATE ET LEUR UTILISATION

(30) Priority: 06.01.2003 US 438585 P
(43) Date of publication of application: 04.11.2015
(62) Divisional of application: 04700403.1
(73) Proprietor: CORIXA CORPORATION, Seattle, WA 98101 (US)
(72) Inventor: Johnson, David A., Hamilton, MT Montana 59840 (US)
(74) Representative: HGF Limited

(56) References cited:
- WO-A2-01/34617
- US-A- 5 597 573
- US-B1- 6 355 257
- PERSING D H ET AL: "TAKING TOLL: LIPID A MIMETICS AS ADJUVANTS AND IMMUNOMODULATORS", TRENDS IN MICROBIOLOGY, ELSEVIER SCIENCE LTD., KIDLINGTON, GB, vol. 10, no. 10, SUPPL, 1 January 2002 (2002-01-01), pages S32-S37, XP009067296, ISSN: 0966-842X, DOI: 10.1016/S0966-842X(02)02426-5
- BAZIN H G ET AL: "The 'Ethereal' nature of TLR4 agonism and antagonism in the AGP class of lipid A mimetics", BIOORGANIC & MEDICINAL CHEMISTRY LETTERS, PERGAMON, AMSTERDAM, NL, vol. 18, no. 20, 15 October 2008 (2008-10-15), pages 5350-5354, XP025562059, ISSN: 0960-894X, DOI: 10.1016/J.BMCL.2008.09.060 [retrieved on 2008-09-19]

## Description

### CROSS-REFERENCE TO RELATED APPLICATIONS

This application claims the priority of U.S. provisional application 60/438,585 filed January 6, 2003, the contents of which are hereby incorporated herein in their entirely.

### BACKGROUND OF THE INVENTION

Toll-like receptors (TLRs), have been linked to the potent innate immune response and recognize distinct structural components that are unique to pathogens; this interaction drives the immune system into an activated state, with short- and long-term consequences. There is significant interest in developing agonists and antagonists of TLRs since the pharmacological manipulation of innate immune responses may lead to more effective vaccines and novel therapeutic approaches to autoimmune, atopic, malignant and infectious diseases. The first microbial product discovered to be a Toll-like receptor agonist was LPS, a bacterial membrane component specific to gram negative bacteria, which activates Toll-like receptor 4 (TLR-4). Although LPS is a potent immunomodulatory agent, its medicinal use is limited due to its extreme toxicity, including the induction of systemic inflammatory response syndrome. The biologically active endotoxic sub-structural moiety of LPS is lipid-A, a phosphorylated, multiply fatty-acid-acylated glucosamine disaccharide that serves to anchor the entire structure in the outer membrane of Gram-negative bacteria. The toxic effects of lipid A can be ameliorated by selective chemical modification of lipid A to produce monophosphoryl lipid A compounds (MPL^{™} immunostimulant; Corixa Corporation; Seattle, WA). Methods of making and using MPL^{™} immunostimulant and structurally like compounds in vaccine adjuvant and other applications have been described (see, for example, U.S. Patent Nos. 4,436,727; 4,877,611; 4,866,034 and 4,912,094; 4,987,237; Johnson et al., J Med Chem 42:4640-4649 (1999); Ulrich and Myers, in Vaccine Design: The Subunit and Adjuvant Approach; Powell and Newman, Eds.; Plenum: New York, 495-524, 1995). In particular, these and other references demonstrated that MPL™ immunostimulant and related compounds had significant adjuvant activities when used in vaccine formulations with protein and carbohydrate antigens for enhancing humoral and/or cell-mediated immunity to the antigens and interact with Toll-like receptors.

Drawing from experience with MPL^{™} immunostimulant and other bacterial cell wall components, a family of novel synthetic compounds, the aminoalkyl glucosaminide phosphates (AGPs), were developed. AGP compounds also interact with TLR-4, as agonists and antagonists. AGPs include both acyclic and cyclic compounds (U.S. Patent Nos. 6,113,918, and 6,303,347, WO 98/50399 published October 12, 1998, WO 01/34617, published May 17, 2001, WO 01/90129, published November 29, 2001, and WO 02/12258, published February 14, 2002). Like MPL^{™} immunostimulant, these compounds have been demonstrated to retain significant adjuvant characteristics when formulated with antigens in vaccine compositions and, in addition, have similar or improved toxicity profiles when compared with MPL™ immunostimulant. AGPs also demonstrate mucosal adjuvant activity and are effective in the absence of antigen, making them attractive compounds for the prophylactic and/or therapeutic use.

Another significant advantage offered by the AGPs over MPL™ immunostimulant and the like is that the AGPs are readily producible on a commercial scale by synthetic means. Since they are produced synthetically AGPs are free of trace biological contaminants found in MPL. As such AGPs would have an advantage over MPL as vaccine adjuvants in certain settings, such as in pediatric immunization protocols where adjuvant pyrogenicity must be minimized. However, because AGPs are chemically synthesized, less then optimum compound stability may lead to the accumulation of degradation products that may result in variable biological activity and stability from lot-to-lot. From the standpoint of developing GMP processes for manufacturing of materials for human clinical trials, lot stability and lot-to-lot variability are major issues. Therefore, compounds that have increased biological activity in comparison to MPL™ immunostimulant and the like, interact with toll-like receptors and/or are optimized for large scale GPL synthesis are desirable. The present invention addresses these needs and more by providing compounds modified to enhanced biological activity, stability with increased resistance to enzymatic and chemical degradation, and/or improved safety profiles.

### SUMMARY OF THE INVENTION

In one aspect, this invention comprises certain novel aminoalkyl glucosaminide phosphate compounds, as defined herein, and pharmaceutically acceptable salts thereof.

### DETAILED DESCRIPTION OF THE INVENTION

The compounds of the subject invention are members of the aminoalkyl glucosaminide 4-phosphate (AGP) family. As described, below, the compounds of the invention variously possess modifications to the lengths of the six acyl chains (primary and secondary), structural modifications of the alkyl arm to include a phosphate moiety, structural modification to include a primary ether lipid at the C-3 sugar position as well as three secondary ether lipids, and/or a 6-hydroxyl blocking group.

Known chemically as ω-aminoalkyl 2-amino-2-deoxy-4-phosphono-β-D-glucopyranosides the AGPs are a class of synthetic lipid A mimetics that are structurally related to the major biologically active component of component in monophosphoryl lipid A. In AGPs the reducing sugar has been replaced with an N-[(R)-3-n-alkanoyloxytetradecanoyl]aminoalkyl aglycon unit. Like other disaccharide lipid A derivatives, the AGPs comprise six fatty acids for maximal biological activity, but unlike disaccharide derivatives, the AGPs contain a conformationally flexible β-linked aglycon unit which permits energetically favored close packing of the six fatty acyl chains. Tight packing of six fatty acids in a hexagonal array is believed to play an essential role in the bioactivity of lipid A-like molecules (Seydel et al., Immunobiol; 187(3-5):191-211, 1993).

The compounds of the present invention are considered to be members of the AGP family. These compounds include modifications to the lengths of the six acyl chains (primary and secondary).

Compounds of the invention are (R)-3-alkyloxytetradecanoic acid derivatives. R₁, R₂ and R₃ are not acyl groups but are straight chain alkyl groups, making the groups R₁O-, R₂O- and R₃O- ether rather than carboxylic acid derivatives. In compounds of formula (III), R₁, R₂ and R₃ are preferably C₆-C₁₀ alkyl groups. They may be the same or different groups, but most preferably are identical.

Such compounds have the general formula (III): 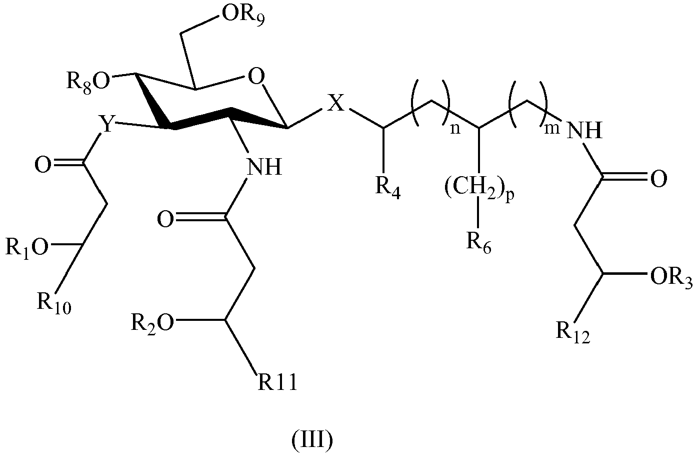 wherein X is selected from the group consisting of O and S at the axial or equatorial position; Y is selected from the group consisting of O and NH; n and m are 0; R₁, R₂ and R₃ are the same or different and are straight chain alkyl groups having from 1 to about 20 carbon atoms and where one of R₁, R₂ or R₃ is optionally hydrogen; R₄ is selected from the group consisting of H and methyl; p is 1 and R₆ is COOH or p is 2 and R₆ is OPO₃H_{2;} R₈ and R₉ are the same or different and are selected from the group consisting of phosphono and H, and at least one of R₈ and R₉ is phosphono; and R₁₀, R₁₁ and R₁₂ are independently selected from straight chain unsubstituted saturated aliphatic groups having from 1 to 11 carbon atoms;
or a pharmaceutically acceptable salt thereof.

In preferred embodiments of compounds (III) of the invention,
- X and Y are preferably both oxygen atoms;
- R₁, R₂ and R₃ are most preferably independently selected from unsubstituted C₆ - C₁₀ straight chain alkyl groups;
- groups R₁₀, R₁₁ and R₁₂ are preferably unsubstituted saturated aliphatic (i.e.,
alkyl) groups having from 1 to 11, preferably from 3 to 9, more preferably from 3 to 7, carbon atoms, and most preferably are identical unsubstituted saturated aliphatic groups having from 3 to 7 carbon atoms.

Yet another type of compound of this invention has the formula (IV): 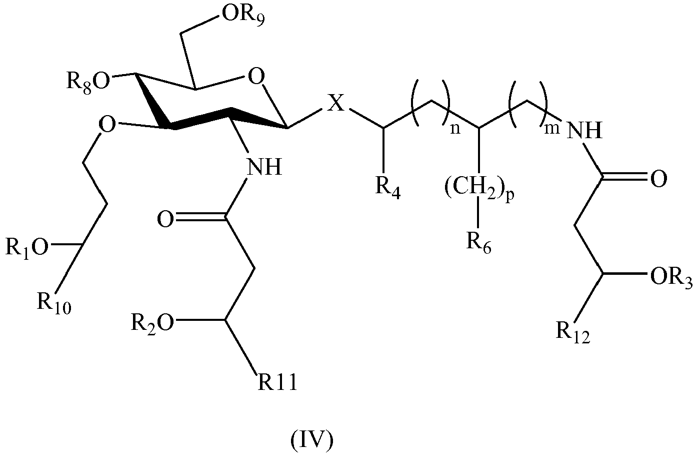 wherein Y is now fixed as oxygen; X is selected from the group consisting of O and S at the axial or equatorial position; n and m are 0; R₁, R₂ and R₃ are the same or different and are straight chain alkyl groups having from 1 to about 20 carbon atoms and where one of R₁, R₂ or R₃ is optionally hydrogen; R₄ is selected from the group consisting of H and methyl; p is 1 and R₆ is COOH or p is 2 and R₆ is OPO₃H_{2;} R₈ and R₉ are the same or different and are selected from the group consisting of phosphono and H, and at
least one of R₈ and R₉ is phosphono; and R₁₀, R₁₁ and R₁₂ are independently selected from straight chain unsubstituted saturated aliphatic groups having from 1 to 10 carbon atoms; or a pharmaceutically acceptable salt thereof.

These compounds thus have two acylated chains and one non-acylated ether chain.

In preferred embodiments of compounds (IV) of the invention,
- X is preferably oxygen;
- R₁, R₂ and R₃ are most preferably independently selected from unsubstituted C₆ - C₁₀ straight chain alkyl groups;
- groups R₁₀, R₁₁ and R₁₂ are preferably unsubstituted saturated aliphatic (i.e.,
alkyl) groups having from 1 to 10, preferably from 3 to 9, more preferably from 3 to 7, carbon atoms, and most preferably are identical unsubstituted saturated aliphatic groups having from 3 to 7 carbon atoms.

These compounds have attributes that allow resistance to unfavorable metabolism and/or aqueous hydrolysis. The selective removal of the normal fatty acids in structurally diverse lipid A molecules by human acyloxyacyl hydrolase (AOAH) to yield the antagonist lipid IVa has been postulated to have evolved as a defense mechanism to reduce lipid A toxicity (Erwin and Munford., J Biol Chem 265(27):16444-16449, 1990). However, the greater toxicity of naturally derived 3-D-MPL relative to that of the major hexaacyl component is likely due to the presence of less highly acylated components with structures distinct from lipid IVa (Ulrich and Myers, Monophosphoryl lipid A as an Adjuvant. Past experiences and new directions. In: Vaccine Design: The Subunit and Adjuvant Approach. Ed. Powell M.F., Newman M.J. Plenum Press, New York, 1995; p. 495-524, Johnson et al., J Med Chem; 42:4640-4649, 1999). The structural variability in 3-D-MPL and other lipid A preparations arises inherently from the cognate LPS as well as from ester cleavage during semi-synthetic and isolation procedures. In fact, it has been reported that facile hydrolytic cleavage of ester-linked acyl groups during the chemical synthesis of a putative *R. capsulatus* lipid A, a potent antagonist of LPS-induced TNF-α production, produces minor amounts of undesirable agonistic by-products (Christ et al., Science; 268:80-83, 1995). Thus, chemical and/or enzymatic instability can be the Achilles' heel of a potential lipid A-based drug containing labile ester linkages. The chemical and metabolic instability of ester-linked fatty acids present in both lipid A agonist and antagonist molecules has been overcome with hydrolytically stable analogs bearing ether linkages in place of primary and/or secondary ester-linked fatty acids (Christ et al. supra, Lien et al., J Biol Chem; 276(3):1873-1880,2001).

The term "protecting group" (represented here by "PG") refers to any of a large number of groups used to replace the hydrogen of a hydroxy group, so as to block, prevent, or reduce reactivity of the group. Examples of protecting groups (and a listing of commonly used abbreviations for them) can be found in T. W. Greene and P. G. Futs, "Protective Groups in Organic Chemistry" (Wiley), Beaucage and Iyer, Tetrahedron 48:2223 (1992) and Harrison et al., Compendium of Synthetic Organic Methods, vols. 1-8 (Wiley). Representative hydroxy protecting groups include those where the hydroxy group is either acylated or alkylated, such as by the formation of ethers or esters using, for instance, methyl, acetyl, benzyl, trityl, alkyl, tetrahydropyranyl, allyl and trisubstituted silyl groups, or in which the hydroxy group is replaced by fluorine.

The choice of a protecting group for a given compound, purpose or set of conditions is within the skill of those in the art, and is done so as to protect, generally or selectively, the reactive group in question under the prevailing conditions (presence of other reactive compounds, pH, temperature, etc.). Protecting groups that may be used in this invention include methyl, phthaloyl, acetyl (Ac), benzyl (Bn), 2,2,2-trichloroethoxycarbonyl (Troc), t-butyldimethylsilyl (TBS), t-butyldiphenylsilyl
(TBDPS), and 2,2,2-trichloro-1,1-dimethylethyl chloroformyl (TCBOC) groups. A fluorine atom may also be used as a protecting group. As is known in the art, a certain protecting group or type of group may be more suitable than others for use with a particular compound or in a given situation, and advantage is taken of these suitabilities in developing processes that involve compounds with reactive groups such as hydroxy.

As discussed herein, the term "aliphatic" by itself or as part of another substituent, means, unless otherwise stated, a straight or branched chain, or cyclic hydrocarbon radical, or combination thereof, which may be fully saturated, mono- or polyunsaturated and can include di- and multivalent radicals, having the number of carbon atoms designated (*i.e.,* C₁-C₁₀ means one to ten carbons). Examples of saturated hydrocarbon radicals include groups such as methyl, ethyl, n-propyl, isopropyl, n-butyl, t-butyl, isobutyl, sec-butyl, cyclohexyl, (cyclohexyl)methyl, cyclopropylmethyl, homologs and isomers of, for example, n-pentyl, n-hexyl, n-heptyl, n-octyl, and the like. An unsaturated aliphatic group is one having one or more double bonds or triple bonds. Examples of unsaturated aliphatic groups include vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. Typically, an aliphatic group will have from 1 to 24 carbon atoms. A "lower aliphatic" group is a shorter chain aliphatic group, generally having eight or fewer carbon atoms.

The term "acyl" refers to a group derived from an organic acid by removal of the hydroxy group. Examples of acyl groups include acetyl, propionyl, dodecanoyl, tetradecanoyl, isobutyryl, and the like. Accordingly, the term "acyl" as used herein is meant to include a group otherwise defined as -C(O)-aliphatic, where the aliphatic group is preferably a saturated aliphatic group.

The term "pharmaceutically acceptable salts" is meant to include salts of the active compounds which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galacturonic acids and the like (see, for example, Berge, S.M., et al, "Pharmaceutical Salts," Journal of Pharmaceutical Science, 66, 1-19, 1977). Certain specific compounds of the present invention contain both basic and acidic functionalities that allow the compounds to be converted into either base or acid addition salts.

The neutral forms of the compounds may be regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

In addition to salt forms, the present invention provides compounds which are in a prodrug form. Prodrugs of the compounds described herein are those compounds that readily undergo chemical changes under physiological conditions to provide the compounds of the present invention. Additionally, prodrugs can be converted to the compounds of the present invention by chemical or biochemical methods in an *ex vivo* environment. For example, prodrugs can be slowly converted to the compounds of the present invention when placed in a transdermal patch reservoir with a suitable enzyme or chemical reagent.

Certain compounds of the present invention can exist in unsolvated forms as well as solvated forms, including hydrated forms. In general, the solvated forms are equivalent to unsolvated forms and are intended to be encompassed within the scope of the present invention. Certain compounds of the present invention may exist in multiple crystalline or amorphous forms. In general, all physical forms are equivalent for the uses contemplated by the present invention and are intended to be within the scope of the present invention.

Certain compounds of the present invention possess asymmetric carbon atoms (optical centers) or double bonds; the racemates, diastereomers, geometric isomers and individual isomers are all intended to be encompassed within the scope of the present invention.

The compounds of the present invention may also contain unnatural proportions of atomic isotopes at one or more of the atoms that constitute such compounds. For example, the compounds may be radiolabeled with radioactive isotopes, such as for example tritium (³H), iodine-125 (¹²⁵I) or carbon-14 (¹⁴C). All isotopic variations of the compounds of the present invention, whether radioactive or not, are intended to be encompassed within the scope of the present invention.

The compounds of the present invention can be prepared by any suitable means; see the Example section below, many of which have been described. For example, processes for preparing certain compounds useful in the present invention are described in U.S. Patent No. 6,113,918; U.S. Patent No. 6,303,347; and PCT/US98/09385 (WO 98/50300, October 12, 1998). Still other compounds can be prepared using methods outlined in Johnson, et al., J. Med. Chem. 42:4640-4649 (1999), Johnson, et al., Bioorg. Med. Chem. Lett. 9:2273-2278 (1999), and PCT/US98/50399 (WO 98/50399, November 12, 1998). In general, the synthetic methods described in the above-noted references and other synthetic methods otherwise familiar in the art are broadly applicable to the preparation of these compounds. For example, in making compounds having different acyl groups and substitutions, one of skill in the art will appreciate that the convergent methods described therein can be modified to use alternate acylating agents, or can be initiated with commercially available materials having appropriate acyl groups attached.

In compositions for eliciting or enhancing an immune response, the compounds of the subject invention are administered to a warm-blooded animal, including humans, with an antigen such as a protein or polypeptide antigen or a polynucleotide that expresses a protein or polypeptide antigen. The amount of antigen administered to elicit a desired response can be readily determined by one skilled in the art and will vary with the type of antigen administered, route of administration and immunization schedule.

Compounds of the present invention can also be administered without an exogenous antigen, to elicit immediate protection via a non-specific resistance effect, as described below; *see* Persing *et al.,* WIPO Publication WO 01/90129, November 29, 2001. Compounds having the ability to stimulate non-specific resistance and/or elicit an adjuvant effect can be used in rapid vaccine formulation. Administration of compounds of the present invention with antigen leads to an acquired mucosal immune response within three to four weeks. Weekly administration of such compounds, via an intranasal route for example, over a four-week period would provide rapid and durable protection by combining the protection provided by the initial innate immune response, followed by the acquired immune response to the antigen of interest.

The compounds of the present invention can be evaluated in a variety of assay formats to identify and select those having the characteristics best suited for a given application of the invention. For example, animal models can be used for identifying and evaluating cytokine release profiles into systemic circulation following administration of a compound of the present invention. In addition, various *in vitro* and *in vivo* models exist for examining changes in one or more aspects of an immune response to different antigenic components in order to identify compounds best suited for eliciting a specific immune response of interest. For example, a compound can be contacted with target cells, such as macrophages, dendritic cells or Langerhans cells *in vitro,* and elaborated cytokines can be measured. In addition, gene expression arrays can be used to identify specific pathways activated or inhibited by a particular compound of interest.

Cytokine induction/production can be determined using treating human blood and/or cells with compounds of the present invention and measuring induction by ELISA (R & D Systems). Such methods can also be used to determine if induction is Toll receptor-dependent. Cytotoxic T lymphocyte response following administration of the compounds of the present invention is determined by ⁵¹Cr-based cytotoxicity assay. If desired, the inventive compound's performance in this regard can be compared to other compounds known to be functional in this regard, such as lipid A, MPL, AGPs or the like. In addition, the inventive compounds may be evaluated in combination with one or more adjuvant and/or immunomodulator agents to identify synergistic effects (see for example US Patent Nos: 6,303,347 and 6,113,918, and WO 01/90129, published November 29, 2001.

Animal models such as murine influenza challenge model and murine *Listeria monocytogenes* challenge model are useful for assessing adjuvant and immunomodulator activity. Briefly, the compound is administered followed by an influenza or *L. monocytogenes* challenge. The disease index (ruffled fur, hunched posture and labored breathing), weight loss and mortality, in the case of influenza or number of colony forming units in the spleens of treated/nontreated mice, in the case of *L. monocytogenes* are monitored as an indication of protection afforded by the inventive compound administration (see for example, WO 01/90129 published November 29, 2001).

### Pharmaceutical Compositions and Methods

It will be understood that, if desired, the compounds disclosed herein may be administered in combination with other therapeutic modalities, such as antimicrobial, antiviral and antifungal compounds or therapies, various DNA-based therapeutics, RNA-based therapeutics, polypeptide-based therapeutics and/or with other immunoeffectors. In fact, essentially any other component may also be included, given that the additional component(s) do not cause a significant adverse effect upon contact with the target cells or host tissues. The compositions may thus be delivered along with various other agents as required or desired for the specific embodiment(s) of the invention being implemented. Illustratively, the pharmaceutical compositions of the invention can include, or be used in conjunction with, DNA encoding one or more therapeutic proteins, antisense RNAs, ribozymes or the like.

Compounds of the invention and compositions comprising them may be administered together with an antigen, to provide an adjuvant or enhancing effect of the antigen, i.e. to enhance the immune response of the patient or subject. Also, compounds and compositions may be administered in the absence of exogenous antigen, for the therapeutic effect of the compound itself.

Where the compound or composition is administered without exogenous antigen, provided are methods for treating, ameliorating and/or substantially preventing infectious diseases in eukaryotic subjects, particularly in animals, preferably in humans. Given the importance of TLR-mediated signalling in the innate immune response to microbial challenge, the ability to stimulate such pathways selectively and with minimal toxicity represents a powerful approach for prophylactic and/or therapeutic treatment modalities against a wide range of infectious agents.

The methods described herein are applicable against essentially any type of infectious agent, including bacteria, viruses, parasites, and fungi. Illustratively, the invention is useful for the prophylactic and/or therapeutic treatment of bacterial infections by species from *Pseudomonas, Escherichia, Klebsiella, Enterobacter, Proteus, Serratia, Candida, Staphylococci, Streptococci, Chlamydia, Mycoplasma and numerous others.* Illustrative viral conditions that may be treated in accordance with the invention include those caused, for example, by Influenza viruses, Adenoviruses, parainfluenza viruses, Rhinoviruses, respiratory syncytial viruses (RSVs), Herpes viruses, Cytomegaloviruses, Hepatitis viruses, e.g., Hepatitis B and C viruses, and others. Illustrative fungi include, for example, *Aspergillis, Candida albicans, Cryptococcus neoformans, Coccidioides immitus,* and others.

Described herein are methods for the treatment of subjects, particularly immunocompromised subjects that have developed or are at risk for developing infections, such as nosocomial bacterial and viral infections. About 2 million of the 40 million individuals hospitalized every year develop nosocomial infection during their stay and about 1% of these, or about 400,000 patients, develop nosocomial pneumonia, more than 7000 of which die. This makes nosocomial pneumonia the leading cause of death in hospital-acquired infections. Thus, this embodiment fills a significant need for effective prophylactic approaches in the treatment of nosocomial infections.

Described herein are prophylactic treatments for immunocompromised patients, such as HIV-positive patients, who have developed or are at risk for developing pneumonia from either an opportunistic infection or from the reactivation of a suppressed or latent infection. In 1992, about 20,000 cases of Pneumocystis carinii infections in AIDS patients were reported in the U.S. alone. Additionally, 60-70% of all AIDS patients get P.carinii at some time during their illness. Thus, the present invention in this embodiment provides effective prophylactic methods for this at-risk population.

Described herein are methods for treating other patient populations that may be immunocompromised and/or at risk for developing infectious diseases, including, for example, patients with cystic fibrosis, chronic obstructive pulmonary disease and other immunocompromised and/or institutionalized patients.

Compounds and compositions described herein may be employed (without exogenous antigen) in methods for treating, ameliorating or substantially preventing allergic disorders and conditions, such as sinusitis, chronic rhinosinusitus, asthma, atopic dermatitis and psoriasis. This approach is based at least in part on the ability of the compounds to activate the production of cytokines from target cells that can compete with stereotypic allergic-type cytokine responses characterized by IL-4 production or hyperresponsiveness to IL-4 activity. Administration of certain of the mono- and disaccharide compounds disclosed herein results in IFN-gamma and IL-12 expression from antigen processing and presenting cells, as well as other cells, resulting in down regulation of cytokines associated with allergic responses such as IL-4, 5, 6, 10 and 13.

Compounds and compositions described herein are employed (without exogenous antigen) in methods for treating autoimmune diseases and conditions. The compounds for use in this embodiment will typically be selected from those capable of antagonizing, inhibiting or otherwise negatively modulating one or more Toll-like receptors, particularly Tlr2 and/or Tlr4, such that an autoimmune response associated with a given condition is ameliorated or substantially prevented. Illustratively, the methods provided by this embodiment can be used in the treatment of conditions such as inflammatory bowel disease, rheumatoid arthritis, chronic arthritis, multiple sclerosis and psoriasis.

The compounds of the subject invention also may be used as adjuvants and immunoeffectors which enhance the generation of antibody in immunized animals, stimulate the production of cytokines and stimulate a cell-mediated immune response including a cytotoxic T-lymphocyte response.

In methods, for example, for effecting the immune response of an individual, the compounds and compositions described herein can be formulated with a pharmaceutically acceptable carrier for injection or ingestion. As used herein, "pharmaceutically acceptable carrier" means a medium that does not interfere with the immunomodulatory activity of the active ingredient and is not toxic to the patient to whom it is administered. Pharmaceutically acceptable carriers include oil-in-water or water-in-oil emulsions, aqueous compositions, liposomes, microbeads and microsomes. For example, the carrier may be a microsphere or microparticle having a compound of this invention within the matrix of the sphere or particle or adsorbed on the surface of the sphere or particle. The carrier may also be an aqueous solution or micellar dispersion containing triethylamine, triethanolamine or other agent that renders the formulation alkaline in nature, or a suspension containing aluminum hydroxide, calcium hydroxide, calcium phosphate or tyrosine adsorbate. Carriers may also include all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated.

Formulations of the compounds of the subject invention that can be administered parenterally, i.e. intraperitoneally, subcutaneously or intramuscularly include the following preferred carriers. Examples of preferred carriers for subcutaneous use include a phosphate buffered saline (PBS) solution and 0.01-0.1 % triethanolamine in USP Water for Injection. Suitable carriers for intramuscular injection include 10% USP ethanol, 40% propylene glycol and the balance an acceptable isotonic solution such as 5% dextrose.

Examples of preferred carriers for intravenous use include 10% USP ethanol, 40% USP propylene glycol and the balance USP Water for Injection. Another acceptable carrier includes 10% USP ethanol and USP Water for Injection; yet another acceptable carrier is 0.01-0.1% triethanolamine in USP Water for Injection. Pharmaceutically acceptable parenteral solvents are such as to provide a solution or dispersion may be filtered through a 5 micron filter without removing the active ingredient.

A preferred method of administration of the compositions of this invention is mucosal administration, particularly intranasal administration or administration by inhalation (pulmonary administration). Pulmonary drug delivery can be achieved by several different approaches, including liquid nebulizers, aerosol-based metered dose inhalers (MDIs), and dry powder dispersion devices. Compositions for use in administrations of this type are typically dry powders or aerosols. For administration of aerosols, which is the preferred method of administration of this invention, the compositions are delivered by inhalers, some types of which are described below.

Dry powders contain, in addition to the active ingredient, a carrier, an absorption enhancer, and optionally other ingredients. The carrier is, for example, a mono-, di- or polysaccharide, a sugar alcohol or another polyol. Suitable carriers include lactose, glucose, raffinose, melezitose, lactitol, maltitol, trehalose, sucrose, mannitol; and starch. Lactose is particularly preferred, especially in the form of its monohydrate. Also included are absorption enhancers such as polypeptides, surfactants, alkyl glycosides, amine salts of fatty acids or phospholipids. The ingredients of the formulation typically must be in a finely divided form, i.e. their volume median diameter should generally be from about 30 to about 200 microns, as measured by a laser diffraction instrument or a coulter counter. The desired particle size may be produced using methods known in the art, e.g. milling, micronization or direct precipitation.

The intranasal route of administration provides numerous advantages over many other forms of administration for the compounds of this invention. For instance, one advantage of intranasal administration is convenience. An injectable system requires sterilization of the hypodermic syringe and in the institutional setting, leads to concerns among medical personnel about the risk of contracting disease by being accidentally stuck by a contaminated needle. Strict requirements for the safe disposal of the used needle and syringe must also be imposed in the institutional setting. In contrast, intranasal administration requires little time on the part of the patient and the attending medical personnel, and is far less burdensome on the institution than injectables.

A second important advantage of intranasal administration is patient acceptance of the drug delivery system. Intranasal administration is perceived as non-invasive, is not accompanied by pain, has no significant after-effects and produces the gratification of prompt relief in the patient exhibiting the symptom. This is of particular advantage when the patient is a child. Another important consideration is that the patient may be able to self-administer the prescribed dosage(s) of nasal spray.

For intranasal administration the compositions of this invention may be formulated as liquids or as solids. Such compositions may contain one or more adjuvants, agents for enhancing absorption of the active ingredients by permeation across the nasal membrane, and (for liquid compositions) an aqueous diluent, for instance water. Alternatively, the diluent may comprise an aqueous buffer such as phosphate buffer. The composition may further optionally include one or more polyhydric alcohols and one or more preservative agents such as, for example, gentamicin, bacitracin (0.005%), or cresol. The compositions may be administered to the nasal cavity in the form of a spray by using an atomizer, nebulizer, sprayer, dropper or other device which insures contact of the solution with the nasal mucous membrane. The device may be a simple one such as a simple nasal sprayer that may be used by the patient, or may be a more elaborate instrument for more accurate dispensing of the compositions, that may be used in a physician's office or a medical facility.

Nasal powder compositions can be made by mixing the active agent and the excipient, both possessing the desired particle size. Firstly, a solution of the active agent and the cyclodextrin excipients made, followed by precipitation, filtration and pulverization. It is also possible to remove the solvent by freeze drying, followed by pulverization of the powder in the desired particle size by using conventional techniques, known from the pharmaceutical literature. The final step is size classification for instance by sieving, to get particles that are preferably between 30 and 200 microns in diameter. Powders can be administered using a nasal insufflator, or they may be placed in a capsule set in an inhalation or insufflation device. A needle is penetrated through the capsule to make pores at the top and the bottom of the capsule and air is sent to blow out the powder particles. Powder formulation can also be administered in a jet-spray of an inert gas or suspended in liquid organic fluids.

The pharmaceutical composition can be delivered in a controlled or sustained release system. In one embodiment, a pump may be used to achieve a controlled or sustained release (see Langer, Science, 249:1527-1533 (1990); Sefton, 1987, CRC Crit. Ref. Biomed. Eng. 14:10; Buschwald et al., 1980, Surgery 88:507; Saudek et al., 1989 N. Engl. J. Med. 321:574). In another embodiment, polymeric materials can be used to achieve controlled or sustained release of the κ-opioid receptor agonist and/or opioid antagonist (see e.g., Medical Applications of Controlled Release, Langer and Wise (eds.), CRC Pres., Boca Raton, Florida 1974); Controlled Drug Bioavailability, Drug Product Design and Performance, Smolen and Ball (eds.), Wiley, New York (1984); Ranger and Peppas, 1983, J. Macromol. Sci. Rev. Macrol. Chem. 23:61; see also Levy et al., 1985 Science 228:190; During et al., 1989, Ann. Neurol. 25:351; Howard et al., 1989, J. Neurosurg. 7 1:105; U.S. Patent No. 5,679,377; U.S. Patent No. 5,916,597, U.S. Patent No. 5,912,015; U.S. Patent No. 5,989,463; U.S. Patent No. 5,128,326; PCT Publication No. WO 99/12154; and PCT Publication No. WO 99/20253). Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polyactides (PLA), poly(lactide-co-glycolides)(PLGA), and polyorthoesters. In a preferred embodiment, the polymer used in a sustained release formulation is inert, free of leachable impurities, stable on storage, sterile, and biodegradable. In yet another embodiment, a controlled or sustained release system can be placed in proximity to the therapeutic target, thus requiring only a fraction of the systematic dose (see, e.g., Goodson, in Medical Applications of Controlled Release, supra, vol. 2, pp. 115-138 (1984)).

Carriers for use with such pharmaceutical compositions are biocompatible, and may also be biodegradable. In certain embodiments, the formulation preferably provides a relatively constant level of active component release. In other embodiments, however, a more rapid rate of release immediately upon administration may be desired. The formulation of such compositions is well within the level of ordinary skill in the art using known techniques. Illustrative carriers useful in this regard include microparticles of poly(lactide-co-glycolide), polyacrylate, latex, starch, cellulose, dextran and the like. Other illustrative delayed-release carriers include supramolecular biovectors, which comprise a non-liquid hydrophilic core (*e.g.,* a cross-linked polysaccharide or oligosaccharide) and, optionally, an external layer comprising an amphiphilic compound, such as a phospholipid (*see e.g.,* U.S. Patent No. 5,151,254 and PCT applications WO 94/20078, WO/94/23701 and WO 96/06638). The amount of active compound contained within a sustained release formulation depends upon the site of implantation, the rate and expected duration of release and the nature of the condition to be treated or prevented.

The compounds of the subject invention are administered to an individual in an effective amount or a pharmaceutically effective amount, to effect or enhance the individual's immune response. As used herein, "effective amount" or "pharmaceutically effective amount" is that amount which shows a response over and above the vehicle or negative controls. An "adjuvant-effective amount" is that amount of the compound in question that, when administered in conjunction with an antigen, shows a response over and above that produced by the antigen alone. The precise dosage of the compounds of the subject invention to be administered to a patient will depend upon the particular compound used, the route of administration, the pharmaceutical composition, and the patient. For example, when administered subcutaneously to enhance an antibody response, the amount of compound used is from 1 to about 250 micrograms, preferably from about 25 to about 50 micrograms based upon administration to a typical 70 kg adult patient.

The pharmaceutical composition is preferably one that induces an immune response predominantly of the Th1 type. High levels of Th1-type cytokines (*e.g.,* IFN-γ, TNFα, IL-2 and IL-12) tend to favor the induction of cell-mediated immune responses to an administered antigen. In contrast, high levels of Th2-type cytokines (*e.g.,* IL-4, IL-5, IL-6 and IL-10) tend to favor the induction of humoral immune responses. Following application of an immunogenic composition as provided herein, a patient will support an immune response that includes Th1- and Th2-type responses. Within a preferred embodiment, in which a response is predominantly Th1-type, the level of Th1-type cytokines will increase to a greater extent than the level of Th2-type cytokines. The levels of these cytokines may be readily assessed using standard assays. Alternatively, or in addition, high levels of Th2-type cytokines (*e.g.,* IL-4, IL-5, IL-6 and IL-10) may be desired for certain therapeutic applications. The levels of these cytokines may be readily assessed using standard assays. For a review of the families of cytokines, see Mosmann and Coffman, Ann. Rev. Immunol. 7:145-173, 1989.

Illustrative compositions for use in induction of Th1-type cytokines include, for example, a combination of CpG-containing oligonucleotides (in which the CpG dinucleotide is unmethylated) as described, for example, in WO 96/02555, WO 99/33488 and U.S. Patent Nos. 6,008,200 and 5,856,462. Immunostimulatory DNA sequences are also described, for example, by Sato et al., Science 273:352, 1996. Other suitable immunostimulants comprise saponins, such as QS21 (Aquila Biopharmaceuticals Inc., Framingham, MA), and related saponin deriviatives and mimetics thereof.

Any of a variety of additional immunostimulants may be included in the compositions of this invention. For example, cytokines, such as GM-CSF, interferons or interleukins to further modulate an immune response of interest. Additionally, Montanide ISA 720 (Seppic, France), SAF (Chiron, California, United States), ISCOMS (CSL), MF-59 (Chiron), the SBAS series of adjuvants (*e.g.,* SBAS-2 or SBAS-4, available from SmithKline Beecham, Rixensart, Belgium), and Enhanzyn™
immunostimulant (Corixa, Hamilton, MT). Polyoxyethylene ether immunostimulants, are described in WO 99/52549A1 and may be used as well.

The pharmaceutical compositions will often further comprise one or more buffers (*e.g.,* neutral buffered saline or phosphate buffered saline), carbohydrates (*e.g.,* glucose, mannose, sucrose or dextrans), mannitol, proteins, polypeptides or amino acids such as glycine, antioxidants, bacteriostats, chelating agents such as EDTA or glutathione, adjuvants (*e.g.,* aluminum hydroxide), solutes that render the formulation isotonic, hypotonic or weakly hypertonic with the blood of a recipient, suspending agents, thickening agents and/or preservatives. Alternatively, compositions of the present invention may be formulated as a lyophilizate.

The pharmaceutical compositions described herein may be presented in unit-dose or multi-dose containers, such as sealed ampoules or vials. Such containers are typically sealed in such a way to preserve the sterility and stability of the formulation until use. In general, formulations may be stored as suspensions, solutions or emulsions in oily or aqueous vehicles. Alternatively, a pharmaceutical composition may be stored in a freeze-dried condition requiring only the addition of a sterile liquid carrier immediately prior to use.

The development of suitable dosing and treatment regimens for using the particular compositions described herein in a variety of treatment regimens, including *e.g.,* oral, parenteral, intravenous, intranasal, and intramuscular administration and formulation, is well known in the art, some of which are briefly discussed below for general purposes of illustration.

In certain applications, the pharmaceutical compositions disclosed herein may be delivered *via* oral administration to an animal. As such, these compositions may be formulated with an inert diluent or with an assimilable edible carrier, or they may be enclosed in hard- or soft-shell gelatin capsule, or they may be compressed into tablets, or they may be incorporated directly with the food of the diet.

The active compounds may even be incorporated with excipients and used in the form of ingestible tablets, buccal tables, troches, capsules, elixirs, suspensions, syrups, wafers, and the like (see, for example, Mathiowitz et al., Nature 1997 Mar 27;386(6623):410-4; Hwang et al., Crit Rev Ther Drug Carrier Sits 1998;15(3):243-84;
U. S. Patent 5,641,515; U. S. Patent 5,580,579 and U. S. Patent 5,792,451). Tablets, troches, pills, capsules and the like may also contain any of a variety of additional components, for example, a binder, such as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar, or both. Of course, any material used in preparing any dosage unit form should be pharmaceutically pure and substantially non-toxic in the amounts employed. In addition, the active compounds may be incorporated into sustained-release preparation and formulations.

Typically, these formulations will contain at least about 0.1% of the active compound or more, although the percentage of the active ingredient(s) may, of course, be varied and may conveniently be between about 1 or 2% and about 60% or 70% or more of the weight or volume of the total formulation. Naturally, the amount of active compound(s) in each therapeutically useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

For oral administration the compositions may alternatively be incorporated with one or more excipients in the form of a mouthwash, dentifrice, buccal tablet, oral spray, or sublingual orally-administered formulation. Alternatively, the active ingredient may be incorporated into an oral solution such as one containing sodium borate, glycerin and potassium bicarbonate, or dispersed in a dentifrice, or added in a therapeutically-effective amount to a composition that may include water, binders, abrasives, flavoring agents, foaming agents, and humectants.
Alternatively the compositions may be fashioned into a tablet or solution form that may be placed under the tongue or otherwise dissolved in the mouth.

In certain circumstances it will be desirable to deliver the pharmaceutical compositions disclosed herein parenterally, intravenously, intramuscularly, or even intraperitoneally. Such approaches are well known to the skilled artisan, some of which are further described, for example, in U. S. Patent 5,543,158; U. S. Patent 5,641,515 and U. S. Patent 5,399,363. Solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations generally will contain a preservative to prevent the growth of microorganisms.

Illustrative pharmaceutical forms suitable for injectable use include sterile aqueous solutions or dispersions and sterile powders for the extemporaneous preparation of sterile injectable solutions or dispersions (for example, see U. S. Patent 5,466,468). In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (*e.g.,* glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and/or by the use of surfactants. The prevention of the action of microorganisms can be facilitated by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For parenteral administration in an aqueous solution, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, a sterile aqueous medium that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. Moreover, for human administration, preparations will of course preferably meet sterility, pyrogenicity, and the general safety and purity standards as required by FDA Office of Biologies standards.

The compositions disclosed herein may be formulated in a neutral or salt form. Illustrative pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective.

The pharmaceutical compositions may be delivered by intranasal sprays, inhalation, and/or other aerosol delivery vehicles. Methods for delivering genes, nucleic acids, and peptide compositions directly to the lungs *via* nasal aerosol sprays has been described, *e.g.,* in U. S. Patent 5,756,353 and U. S. Patent 5,804,212. Likewise, the delivery of drugs using intranasal microparticle resins (Takenaga et al., J Controlled Release 1998 Mar 2;52(1-2):81-7) and lysophosphatidyl-glycerol compounds (U. S. Patent 5,725,871) are also well-known in the pharmaceutical arts. Likewise, illustrative transmucosal drug delivery in the form of a polytetrafluoroetheylene support matrix is described in U. S. Patent 5,780,045.

In certain cases, liposomes, nanocapsules, microparticles, lipid particles, vesicles, and the like, are used for the introduction of the compositions of the present invention into suitable host cells/organisms. In particular, the compositions described herein may be formulated for delivery either encapsulated in a lipid particle, a liposome, a vesicle, a nanosphere, or a nanoparticle or the like. Alternatively, compositions of the present invention can be bound, either covalently or non-covalently, to the surface of such carrier vehicles.

The formation and use of liposome and liposome-like preparations as potential drug carriers is generally known to those of skill in the art (see for example, Lasic, Trends Biotechnol 1998 Jul;16(7):307-21; Takakura, Nippon Rinsho 1998 Mar;56(3):691-5; Chandran et al., Indian J Exp Biol. 1997 Aug;35(8):801-9; Margalit, Crit Rev Ther Drug Carrier Syst. 1995;12(2-3):233-61; U.S. Patent 5,567,434; U.S. Patent 5,552,157; U.S. Patent 5,565,213; U.S. Patent 5,738,868 and U.S. Patent 5,795,587).

Liposomes have been used successfully with a number of cell types that are normally difficult to transfect by other procedures, including T cell suspensions, primary hepatocyte cultures and PC 12 cells (Renneisen et al., J Biol Chem. 1990 Sep 25;265(27):16337-42; Muller et al., DNA Cell Biol. 1990 Apr;9(3):221-9). In addition, liposomes are free of the DNA length constraints that are typical of viral-based delivery systems. Liposomes have been used effectively to introduce genes, various drugs, radiotherapeutic agents, enzymes, viruses, transcription factors, allosteric effectors and the like, into a variety of cultured cell lines and animals. Furthermore, the use of liposomes does not appear to be associated with autoimmune responses or unacceptable toxicity after systemic delivery.

In certain cases, liposomes are formed from phospholipids that are dispersed in an aqueous medium and spontaneously form multilamellar concentric bilayer vesicles (also termed multilamellar vesicles (MLVs).

Alternatively, described herein are pharmaceutically-acceptable nanocapsule formulations of the compositions described herein. Nanocapsules can generally entrap compounds in a stable and reproducible way (see, for example, Quintanar-Guerrero et al., Drug Dev Ind Pharm. 1998 Dec;24(12):1113-28). To avoid side effects due to intracellular polymeric overloading, such ultrafine particles (sized around 0.1 µm) may be designed using polymers able to be degraded *in vivo.* Such particles can be made as described, for example, by Couvreur et al., Crit Rev Ther Drug Carrier Syst. 1988;5(1):1-20; zur Muhlen et al., Eur J Pharm Biopharm. 1998 Mar;45(2):149-55; Zambaux et al. J Controlled Release. 1998 Jan 2;50(1-3):31-40; and U. S. Patent 5,145,684.

### Cancer Therapies

Immunologic approaches to cancer therapy are based on the recognition that cancer cells can often evade the body's defenses against aberrant or foreign cells and molecules, and that these defenses might be therapeutically stimulated to regain the lost ground, *e.g.* pgs. 623-648 in Klein, Immunology (Wiley-Interscience, New York, 1982). Numerous recent observations that various immune effectors can directly or indirectly inhibit growth of tumors has led to renewed interest in this approach to cancer therapy, *e.g.* Jager, et al., Oncology 2001;60(1):1-7; Renner, et al., Ann Hematol 2000 Dec;79(12):651-9.

Four-basic cell types whose function has been associated with antitumor cell immunity and the elimination of tumor cells from the body are: i) B-lymphocytes which secrete immunoglobulins into the blood plasma for identifying and labeling the nonself invader cells; ii) monocytes which secrete the complement proteins that are responsible for lysing and processing the immunoglobulin-coated target invader cells; iii) natural killer lymphocytes having two mechanisms for the destruction of tumor cells, antibody-dependent cellular cytotoxicity and natural killing; and iv) T-lymphocytes possessing antigen-specific receptors and having the capacity to recognize a tumor cell carrying complementary marker molecules (Schreiber, H., 1989, in Fundamental Immunology (ed). W. E. Paul, pp. 923-955).

Cancer immunotherapy generally focuses on inducing humoral immune responses, cellular immune responses, or both. Moreover, it is well established that induction of CD4⁺ T helper cells is necessary in order to secondarily induce either antibodies or cytotoxic CD8⁺ T cells. Polypeptide antigens that are selective or ideally specific for cancer cells offer a powerful approach for inducing immune responses against cancer, and are an important aspect of the present invention.

Therefore, in further aspects of the present invention, the pharmaceutical compositions described herein may be used to stimulate an immune response against cancer. Within such methods, the pharmaceutical compositions described herein are administered to a patient, typically a warm-blooded animal, preferably a human. A patient may or may not be afflicted with cancer. Pharmaceutical compositions and vaccines may be administered either prior to or following surgical removal of primary tumors and/or treatment such as administration of radiotherapy or conventional chemotherapeutic drugs. As discussed above, administration of the pharmaceutical compositions may be by any suitable method, including administration by intravenous, intraperitoneal, intramuscular, subcutaneous, intranasal, intradermal, anal, vaginal, topical and oral routes.

Within certain embodiments, immunotherapy may be active immunotherapy, in which treatment relies on the *in vivo* stimulation of the endogenous host immune system to react against tumors with the administration of immune response-modifying agents (such as polypeptides and polynucleotides as provided herein).

Routes and frequency of administration of the therapeutic compositions described herein, as well as dosage, will vary from individual to individual, and may be readily established using standard techniques. In general, the pharmaceutical compositions and vaccines may be administered by injection (*e.g*., intracutaneous, intramuscular, intravenous or subcutaneous), intranasally (*e.g.*, by aspiration) or orally. Preferably, between 1 and 10 doses may be administered over a 52 week period. Preferably, 6 doses are administered, at intervals of 1 month, and booster vaccinations may be given periodically thereafter. Alternate protocols may be appropriate for individual patients. A suitable dose is an amount of a compound that, when administered as described above, is capable of promoting an anti-tumor immune response, and is at least 10-50% above the basal (*i.e*., untreated) level. Such response can be monitored by measuring the anti-tumor antibodies in a patient or by vaccine-dependent generation of cytolytic effector cells capable of killing the patient's tumor cells *in vitro.* Such vaccines should also be capable of causing an immune response that leads to an improved clinical outcome (*e.g.*, more frequent remissions, complete or partial or longer disease-free survival) in vaccinated patients as compared to non-vaccinated patients. In general, for pharmaceutical compositions and vaccines comprising one or more polypeptides, the amount of each polypeptide present in a dose ranges from about 25 µg to 5 mg per kg of host. Suitable dose sizes will vary with the size of the patient, but will typically range from about 0.1 mL to about 5 mL.

In general, an appropriate dosage and treatment regimen provides the active compound(s) in an amount sufficient to provide therapeutic and/or prophylactic benefit. Such a response can be monitored by establishing an improved clinical outcome (*e.g.,* more frequent remissions, complete or partial, or longer disease-free survival) in treated patients as compared to non-treated patients. Increases in preexisting immune responses to a tumor protein generally correlate with an improved clinical outcome. Such immune responses may generally be evaluated using standard proliferation, cytotoxicity or cytokine assays, which may be performed using samples obtained from a patient before and after treatment.

The present invention is further described by way of the following nonlimiting Examples and Test Examples that are given for illustrative purposes only.

### EXAMPLES

### Reference Example 1

### Primary Fatty Acyl Chain Modifications

This example describes preparation of primary fatty acid derivatives having variable length primary fatty acyl chains, alone or in combination with variable secondary fatty acid chains. For example, compounds **1a-c** and **2a-c,** in which short (C₆) and medium (C₁₀) chain primary fatty acids are combined with short, medium, or long chain secondary fatty acids.

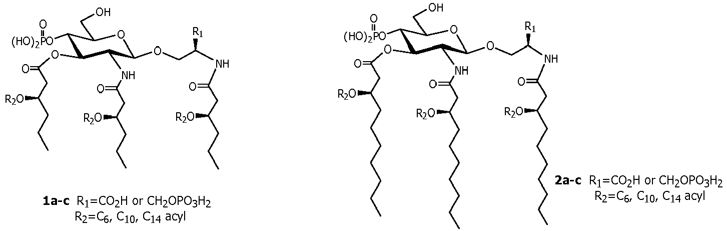

These compounds are prepared either using the well-established serine aglycon (R₁=CO₂H) or, alternatively, using a chemically more stable and ionizable serinol phosphate aglycon unit (R₁=CH₂OPO₃H₂). The seryl/serinol phosphate selection will be based on comparison of the biological activities of known seryl derivatives **3a,b** with novel serinol phosphates **4a,b.**

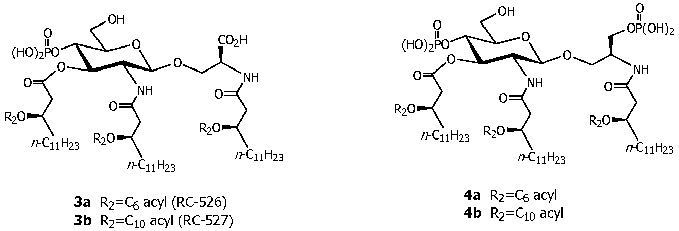

The primary chain-modified compounds derivatives are synthesized by a modification of a previously described method (B1 in Johnson et al., US Patent No. 6,355,251) employing a common advanced intermediate that allows introduction of the amide- and ester-linked acyloxy acids near the end of the synthesis (Scheme I). The initial step in the synthesis is glycosylation of acceptor **6** with the known tetraacetate **5** (prepared in 4 steps from glucosamine) to give β-glycoside **7** and conversion of **7** to common advanced intermediate (CAI) **8,** which is optimized for R₁=CO₂Bn. Selective 4-O-acylation and N-deprotection/acylation results in hexaacyl derivative **9,** which is converted in 3-4 steps to **1a-c** or **2a-c** via phosphorylation and deblocking.

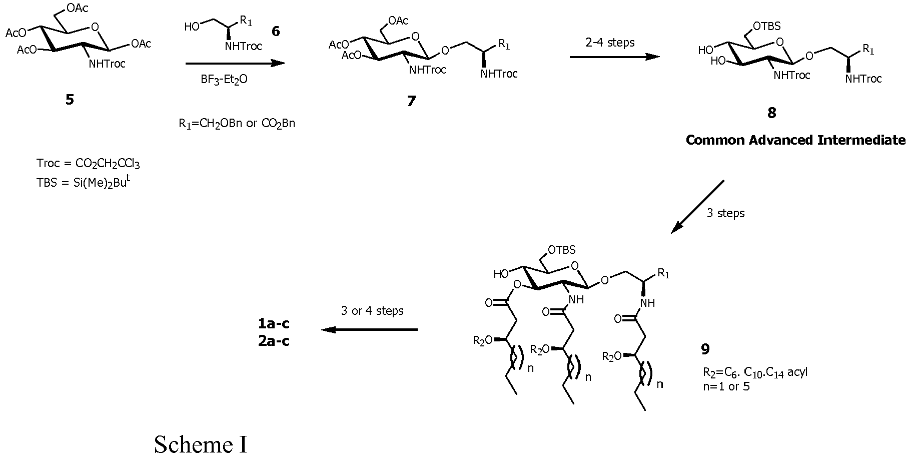

The requisite (R)-3-*n*-alkanoyloxyalkanoic acids are prepared according to Keegan et al., Tetrahedron: Asymmetry; 7(12):3559-3564, 1996, starting with the appropriate 3-oxo methyl esters. High chemical and diastereomeric purity of the products **1** and **2** is achieved by either normal phase chromatography on silica gel or, alternatively, via cellulose chromatography or liquid-liquid partition chromatography on Sephadex LH-20 gel. The purity of the isolated triethylammonium salts is established by spectroscopic (IR, ¹H and ¹³C NMR) and physical (combustion analysis, FAB-MS) means, as well as by HPLC.

### Reference Example 2

### Glycol and phosphonooxyethyl (PE) Compounds

This example describes the synthesis of glycyl compounds **11a,b** and phosphonooxyethyl (PE) compounds **12a,b,** which are nearly regioisomeric with **3a,b** and **4a,b.**

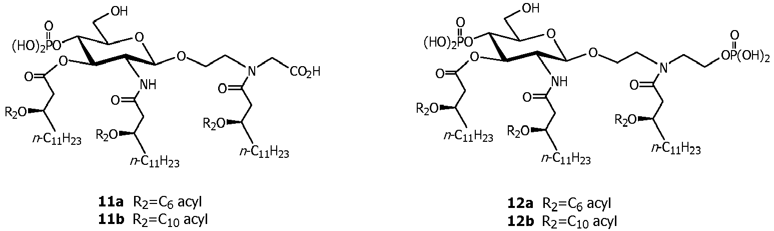

These compounds are most easily prepared by a more convergent synthesis than that outlined in Scheme I, in which a common C₆ or C₁₀ glycosyl donor **13** is coupled with an appropriate N-acylated (or, alternatively, N-Troc protected - not shown) acceptor unit **14** or **15** in the presence of silver ion to give β-glycosides **16** (Scheme II). The glycine acceptor **14** is prepared according to Bulusu et a., J Med Chem; 35(19):3463-3469, 1992 from ethanolamine and benzyl (or t-butyl) bromoacetate followed by N-acylation or protection. The phosphate **15** is prepared by monophosphorylation of N-acylated (or protected) diethanolamine. N-deprotection/acylation or N,N-diacylation in case of Troc-protected aglycon(Jiang et al., Tetrahedron; 58(43):8833-8842, 2002) of the β-glycosides **16** and cleavage of the phenyl and other protecting groups of the resulting hexaacylated derivative **17** is expected to give the desired compounds **11a,b** and **12a,b,** which are isolated and characterized as their triethylammonium salts after chromatographic purification on silica or LH-20 gel or DEAE cellulose.

Compounds **16** are novel and form yet another aspect of this invention.

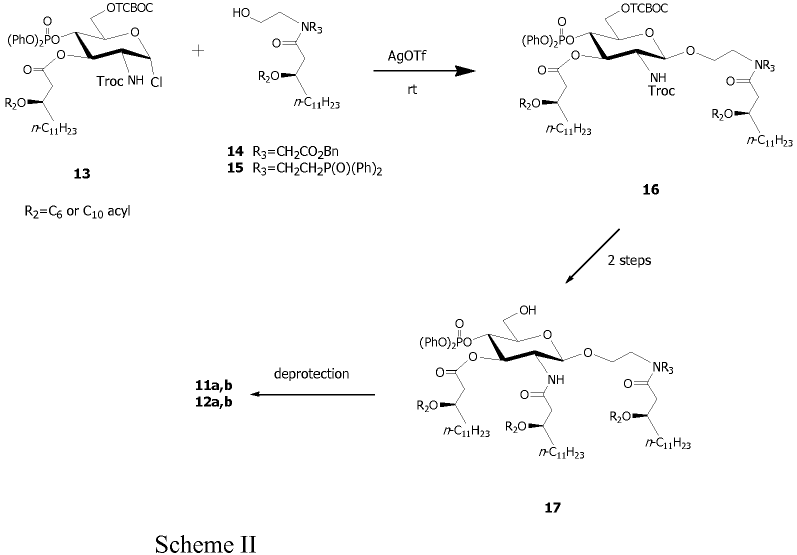

### Reference Example 3

### Secondary ether lipids

This example describes synthesis of (R)-3-alkyloxytetradecanoic acid derivatives **(18a,b)** which are resistant to unfavorable metabolism and/or aqueous hydrolysis. To synthesize compounds **18a,b** the ether lipid analogs of the secondary fatty acids compounds **3a,b** or the corresponding serinol phosphates **5a,b** must be done initially. As shown retrosynthetically in Scheme III, the synthesis of target molecules **18a,b** can be achieved by substituting (R)-3-hexyloxytetradecanoic acid or (R)-3-decyloxytetradecanoic acid for the corresponding acyloxyacids beginning with selective 3-O-acylation of common advanced intermediate **8** in Scheme I and proceeding through intermediate **9** (R₂=C₆ or C₁₀ alkyl, n=9). The requisite alkyloxyacids **19** are synthesized from (R)-3-hydroxytetradecanoic acid or its phenacyl ester, intermediates in the acyloxyacid syntheses, by known methods in >50% overall yield (Keegan et al., Tetrahedron: Asymmetry; 7(12):3559-3564, 1996, Watanabe et al., Carbohydr Res; 332(3):257-277, 2001, Jiang., Bioorg Med Chem Lett; 12(16):2193-2196, 2002, Christ et al., US Patent NO: 5,530,113. 1996).

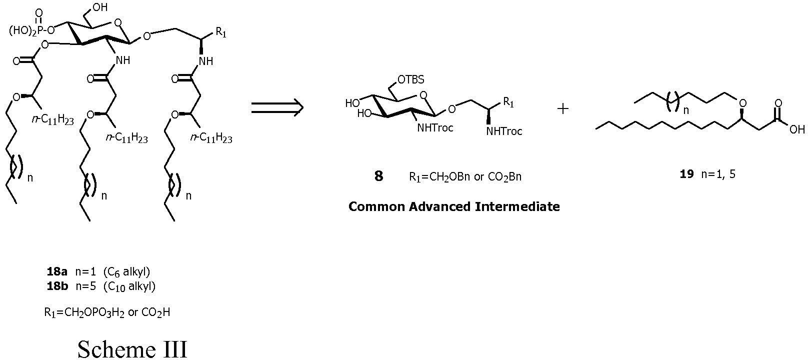

### Reference Example 4

### Primary and secondary ether lipids

This example describes compounds **(20a,b)** containing a primary ether lipid at the C-3 sugar position as well as three secondary ether lipids. These compounds are synthesized by alkylation of acetonide **21,** an intermediate in the synthesis of glycosyl donor **13** (Scheme II), with sulfonate **22,** which in turn is generated in one step from the alcohol precursor of **19,** to give diether **23** (Scheme IV). 4,6-Functionalization and anomeric activation provides glycosyl chloride **24,** which is then processed as in Scheme II using the corresponding alkyloxyacids in the N-acylation steps. In an alternative scheme, the 2-azido⁴² or 2-trifluoroacetamido⁴⁵ derivative can be employed in the 3-O-alkylation step.

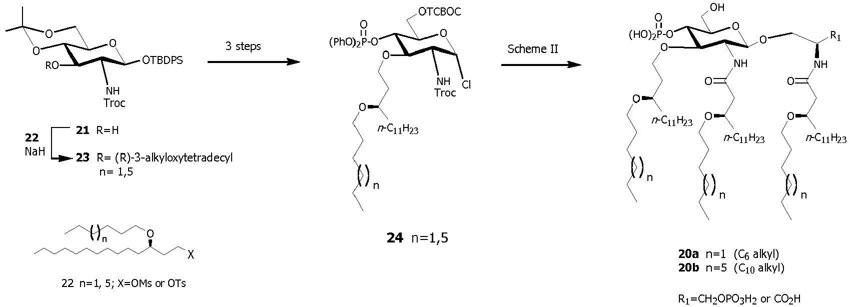

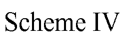

### Reference Example 5

### C-6 modified compounds

This example describes the compounds having a blocked 6-hydroxyl. The this example a methyl ether or a fluoro group is used in conjunction with seryl or serinol phosphate compounds **25a,b** and **26a,b.** As mentioned above, these compounds also form an aspect of the invention.

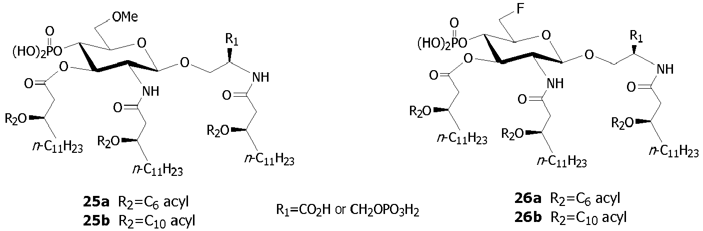

The compounds are prepared from diol **27** as shown in Scheme V. Intermediate **27,** obtained in two steps from acetonide **21,** is functionalized on the 6-position by known methods (Christ et al., US Patent No: 5,530,113, 1996; Watanabe et al., Carbohydr Res; 333(3):203-231, 2001) to give alcohol **28.** Conversion of **28** to the chlorides **29** in two steps and elaboration according to Scheme II provides the target molecules **25a,b** and **26a,b.** Compounds with primary and/or secondary ether linkages as described in Example 4 above can be modified as described in this example to further protect the molecules against chemical and enzymatic degradation.

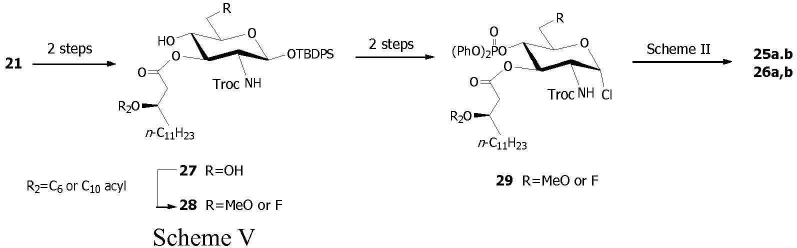

It is understood that the foregoing examples are merely illustrative of the present invention.

## Claims

1. A compound having the formula (III) 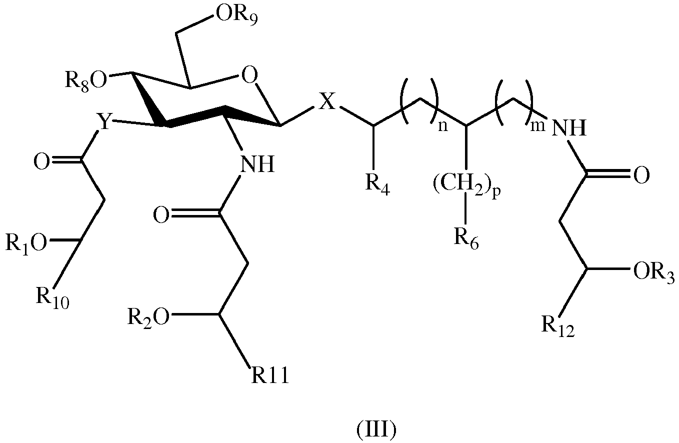 wherein X is selected from the group consisting of O and S at the axial or equatorial position; Y is selected from the group consisting of O and NH; n and m are 0; R₁, R₂ and R₃ are the same or different and are straight chain alkyl groups having from 1 to 20 carbon atoms and where one of R₁, R₂ or R₃ is optionally hydrogen; R₄ is selected from the group consisting of H and methyl; p is 1 and R₆ is COOH or p is 2 and R₆ is OPO₃H₂; R₈ and R₉ are the same or different and are selected from the group consisting of phosphono and H, and at least one of R₈ and R₉ is phosphono; and R₁₀, R₁₁ and R₁₂ are independently selected from straight chain unsubstituted saturated aliphatic groups having from 1 to 11 carbon atoms;
or a pharmaceutically acceptable salt thereof.

2. A compound according to claim 1 wherein X and Y are oxygen, R₁, R₂ and R₃ are independently selected from C₆-C₁₀ straight chain alkyl groups, and R₁₀, R₁₁ and R₁₂ are independently straight chain unsubstituted saturated aliphatic groups having from 3 to 9 carbon atoms.

3. A compound according to claim 2 wherein R₁₀, R₁₁ and R₁₂ are independently straight chain unsubstituted aliphatic groups having from 3 to 7 carbon atoms.

4. A compound having the formula (IV): 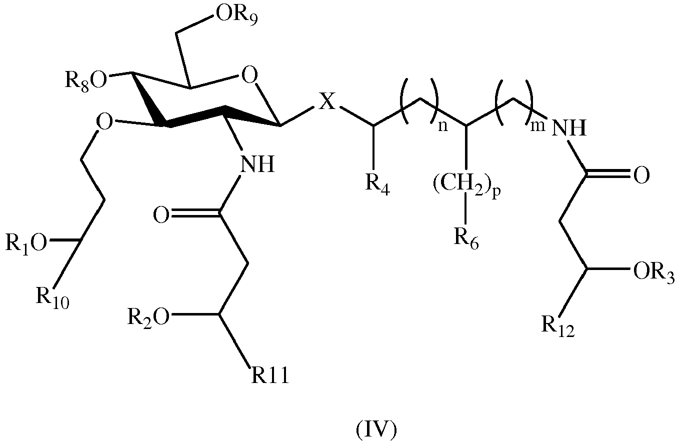 wherein X is selected from the group consisting of O and S at the axial or equatorial position; n and m are 0; R₁, R₂ and R₃ are the same or different and are straight chain alkyl groups having from 1 to 20 carbon atoms and where one of R₁, R₂ or R₃ is optionally hydrogen; R₄ is selected from the group consisting of H and methyl; p is 1 and R₆ is COOH or p is 2 and R₆ is OPO₃H_{2;} R₈ and R₉ are the same or different and are selected from the group consisting of phosphono and H, and at least one of R₈ and R₉ is phosphono; and R₁₀, R₁₁ and R₁₂ are independently selected from straight chain unsubstituted saturated aliphatic groups having from 1 to 10 carbon atoms;
or a pharmaceutically acceptable salt thereof.

5. A compound according to claim 4 wherein X is oxygen, R₁, R₂ and R₃ are independently selected from C₆-C₁₀ straight chain alkyl groups, and R₁₀, R₁₁ and R₁₂ are independently straight chain unsubstituted saturated aliphatic groups having from 3 to 9 carbon atoms

6. A compound according to claim 5 wherein R₁₀, R₁₁ and R₁₂ are independently straight chain unsubstituted aliphatic groups having from 3 to 7 carbon atoms.

7. A pharmaceutical composition comprising an effective amount of a compound according to any one of claims 1 and 4, or a pharmaceutically acceptable salt thereof and a pharmaceutically acceptable carrier.

8. A compound according to any one of claims 1 and 4, or a pharmaceutically acceptable salt thereof, for use in enhancing the immune response of a subject.

## Patentansprüche

1. Verbindung mit der folgenden Formel (III) 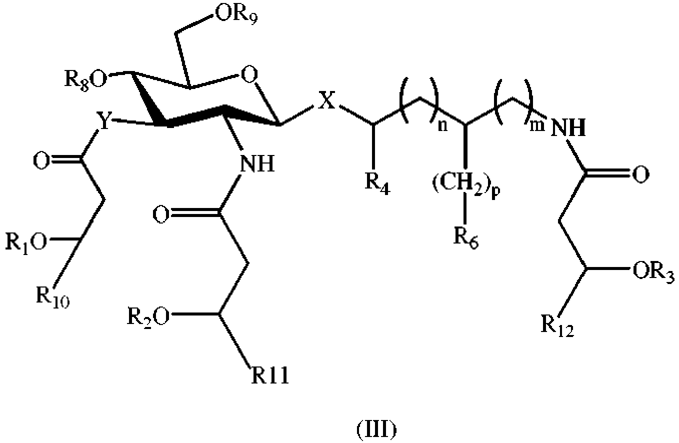 wobei X ausgewählt ist aus der Gruppe bestehend aus O und S an der axialen oder äquatorialen Position; Y ausgewählt ist aus der Gruppe bestehend aus O und NH; n und m 0 sind; R₁, R₂ und R₃ gleich oder verschieden sind und geradkettige Alkyl-Gruppen sind und von 1 bis 20 Kohlenstoffatome haben und wobei eines von R₁, R₂ oder R₃ optional Wasserstoff ist; R₄ ausgewählt ist aus der Gruppe bestehend aus H und Methyl; p 1 ist und R₆ COOH ist, oder p 2 ist und R₆ OPO₃H₂ ist; R₈ und R₉ gleich oder verschieden sind und ausgewählt sind aus der Gruppe bestehend aus Phosphon und H, und mindestens eines von R₈ und R₉ Phosphon ist; und R₁₀, R₁₁ und R₁₂ unabhängig ausgewählt sind aus geradkettigen unsubstituierten gesättigten aliphatischen Gruppen mit von 1 bis 11 Kohlenstoffatomen; oder ein pharmazeutisch annehmbares Salz davon.

2. Verbindung nach Anspruch 1 wobei X und Y Sauerstoff sind, R₁, R₂ und R₃ unabhängig ausgewählt sind aus C₆-C₁₀ geradkettigen Alkyl-Gruppen, und R₁₀, R₁₁ und R₁₂ unabhängig geradkettige unsubstituierte gesättigte aliphatische Gruppen mit von 3 bis 9 Kohlenstoffatomen sind.

3. Verbindung nach Anspruch 2 wobei R₁₀, R₁₁ und R₁₂ unabhängig geradkettige unsubstituierte aliphatische Gruppen mit von 3 bis 7 Kohlenstoffatomen sind.

4. Verbindung mit der folgenden Formel (IV): 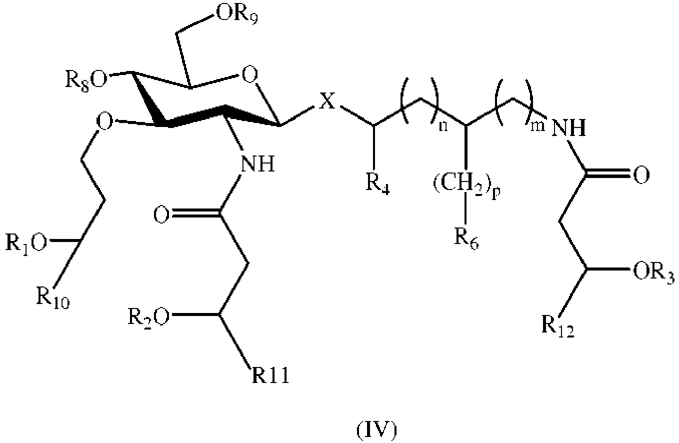 wobei X ausgewählt ist aus der Gruppe bestehend aus O und S an der axialen oder äquatorialen Position; n und m 0 sind; R₁, R₂ und R₃ gleich oder verschieden sind und geradkettige Alkyl-Gruppen sind und von 1 bis 20 Kohlenstoffatome haben und wobei eines von R₁, R₂ oder R₃ optional Wasserstoff ist; R₄ ausgewählt ist aus der Gruppe bestehend aus H und Methyl; p 1 ist und R₆ COOH ist, oder p 2 ist und R₆ OPO₃H₂ ist; R₈ und R₉ gleich oder verschieden sind und ausgewählt sind aus der Gruppe bestehend aus Phosphon und H, und mindestens eines von R₈ und R₉ Phosphon ist; und R₁₀, R₁₁ und R₁₂ unabhängig ausgewählt sind aus geradkettigen unsubstituierten gesättigten aliphatischen Gruppen mit von 1 bis 10 Kohlenstoffatomen; oder ein pharmazeutisch annehmbares Salz davon.

5. Verbindung nach Anspruch 4 wobei X Sauerstoff ist, R₁, R₂ und R₃ unabhängig ausgewählt sind aus C₆-C₁₀ geradkettigen Alkyl-Gruppen, und R₁₀, R₁₁ und R₁₂ unabhängig geradkettige unsubstituierte gesättigte aliphatische Gruppen mit von 3 bis 9 Kohlenstoffatomen sind.

6. Verbindung nach Anspruch 5 wobei R₁₀, R₁₁ und R₁₂ unabhängig geradkettige unsubstituierte aliphatische Gruppen mit von 3 bis 7 Kohlenstoffatomen sind.

7. Pharmazeutische Zusammensetzung umfassend eine wirksame Menge einer Verbindung nach einem der Ansprüche 1 und 4, oder ein pharmazeutisch annehmbares Salz davon und einen pharmazeutisch annehmbaren Träger.

8. Verbindung nach einem der Ansprüche 1 und 4, oder ein pharmazeutisch annehmbares Salz davon, zum Verbessern einer Immunantwort eines Subjekts.

## Revendications

1. Composé représenté par la formule (III) : 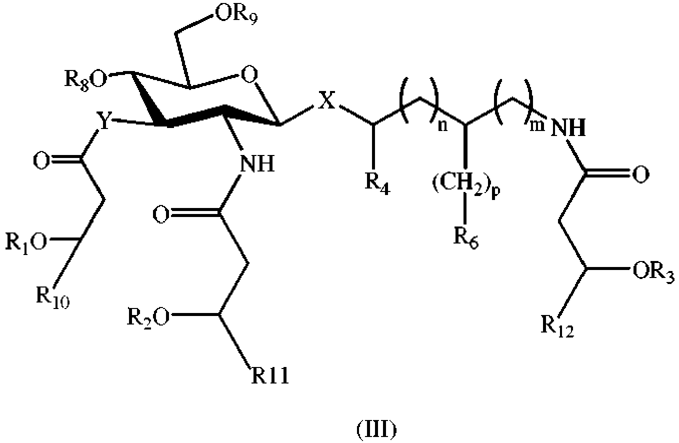 dans laquelle X est choisi dans le groupe constitué par un atome O et un atome S en position axiale ou équatoriale ; Y est choisi dans le groupe constitué par un atome O et un groupe NH ; n et m sont égaux à 0 ; R₁, R₂ et R₃ sont identiques ou différents et représentent des groupes alkyle à chaîne droite comportant de 1 à 20 atomes de carbone et où l'un des groupes R₁, R₂ ou R₃ représente éventuellement un atome d'hydrogène ; R₄ est choisi dans le groupe constitué par un atome H et un groupe méthyle ; p est égal à 1 et R₆ représente un groupe COOH ou p est égale à 2 et R₆ représente un groupe OPO₃H₂ ; R₈ et R₉ sont identiques ou différents et sont choisis dans le groupe constitué par un groupe phosphono et un atome H, et au moins l'un des groupes R₈ et R₉ représente un groupe phosphono ; et R₁₀, R₁₁ et R₁₂ sont indépendamment choisis parmi des groupes aliphatiques saturés non substitués à chaîne droite comportant de 1 à 11 atomes de carbone ;
ou sel pharmaceutiquement acceptable de celui-ci.

2. Composé selon la revendication 1, X et Y représentant un atome d'oxygène, R₁, R₂ et R₃ étant indépendamment choisis parmi des groupes alkyle à chaîne droite en C₆ à C₁₀, et R₁₀, R₁₁ et R₁₂ représentant indépendamment des groupes aliphatiques saturés non substitués à chaîne droite comportant de 3 à 9 atomes de carbone.

3. Composé selon la revendication 2, R₁₀, R₁₁ et R₁₂ représentant indépendamment des groupes aliphatiques non substitués à chaîne droite comportant de 3 à 7 atomes de carbone.

4. Composé représenté par la formule (IV) : 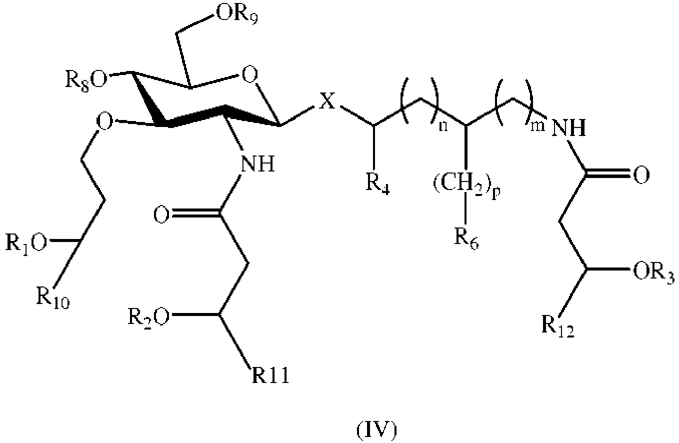 dans laquelle X est choisi dans le groupe constitué par un atome O et un atome S en position axiale ou équatoriale ; n et m sont égaux à 0 ; R₁, R₂ et R₃ sont identiques ou différents et représentent des groupes alkyle à chaîne droite comportant de 1 à 20 atomes de carbone et où l'un des groupes R₁, R₂ ou R₃ représente éventuellement un atome d'hydrogène ; R₄ est choisi dans le groupe constitué par un atome H et un groupe méthyle ; p est égal à 1 et R₆ représente un groupe COOH ou p est égal à 2 et R₆ représente un groupe OPO₃H₂ ; R₈ et R₉ sont identiques ou différents et sont choisis dans le groupe constitué par un groupe phosphono et un atome H, et au moins l'un des groupes R₈ et R₉ représente un groupe phosphono ; et R₁₀, R₁₁ et R₁₂ sont indépendamment choisis parmi des groupes aliphatiques saturés non substitués à chaîne droite comportant de 1 à 10 atomes de carbone ;
ou sel pharmaceutiquement acceptable de celui-ci.

5. Composé selon la revendication 4, X représentant un atome d'oxygène, R₁, R₂ et R₃ étant indépendamment choisis parmi des groupes alkyle à chaîne droite en C₆ à C₁₀, et R₁₀, R₁₁ et R₁₂ représentant indépendamment des groupes aliphatiques saturés non substitués à chaîne droite comportant de 3 à 9 atomes de carbone.

6. Composé selon la revendication 5, R₁₀, R₁₁ et R₁₂ représentant indépendamment des groupes aliphatiques non substitués à chaîne droite comportant de 3 à 7 atomes de carbone.

7. Composition pharmaceutique comprenant une quantité efficace d'un composé selon l'une quelconque des revendications 1 et 4, ou un sel pharmaceutiquement acceptable de celui-ci et un excipient pharmaceutiquement acceptable.

8. Composé selon l'une quelconque des revendications 1 et 4, ou sel pharmaceutiquement acceptable de celui-ci, destiné à être utilisé pour améliorer la réponse immunitaire d'un sujet.
